(19) Europäisches Patentamt
European Patent Office
Office européen des brevets

(11) EP 4 483 792 A1

(12) **EUROPEAN PATENT APPLICATION**

(43) Date of publication:
**01.01.2025 Bulletin 2025/01**

(21) Application number: **23181942.6**

(22) Date of filing: **28.06.2023**

(51) International Patent Classification (IPC):
***A61B 5/021*** (2006.01) ***A61B 5/022*** (2006.01)
***A61B 5/00*** (2006.01)

(52) Cooperative Patent Classification (CPC):
**A61B 5/02141; A61B 5/02233;** A61B 5/02108; A61B 5/02125; A61B 5/6824

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
- **SCHMITT, Lars**
**Eindhoven (NL)**
- **MUEHLSTEFF, Jens**
**Eindhoven (NL)**
- **MENA BENITO, Maria Estrella**
**5656AG Eindhoven (NL)**
- **WIJSHOFF, Ralph Wilhelm Christianus Gemma Rosa**
**Eindhoven (NL)**
- **HILGERS, Achim Rudolf**
**Eindhoven (NL)**
- **PELSSERS, Eduard Gerard Marie**
**Eindhoven (NL)**
- **DAVIDOIU, Valentina-Geta**
**Eindhoven (NL)**

(74) Representative: **Philips Intellectual Property & Standards**
**High Tech Campus 52**
**5656 AG Eindhoven (NL)**

(54) **SYSTEM FOR USE IN HEMODYNAMIC PARAMETER MEASUREMENT**

(57) A system for use in non-invasive hemodynamic parameter measurement which comprises a cuff having a pressure actuator and a tissue pressure sensor, where the tissue pressure sensor comprises a fluid pad hydraulically connected to a fluid pressure transducer unit. Provided is a height monitoring means for tracking a change in a height of the transducer unit relative to the cuff and the cuff relative to a heart level of the patient, wherein the heart level is indicated by proxy using a height reference unit mounted to the patient's torso. Changes in these relative heights are used to compute a correction parameter for the tissue pressure signal. In some embodiments, changes in height of each of the cuff, transducer unit and height reference unit may be independently tracked. In some embodiments, this is done by motion tracking of each unit and aggregating net motion to determine position change.

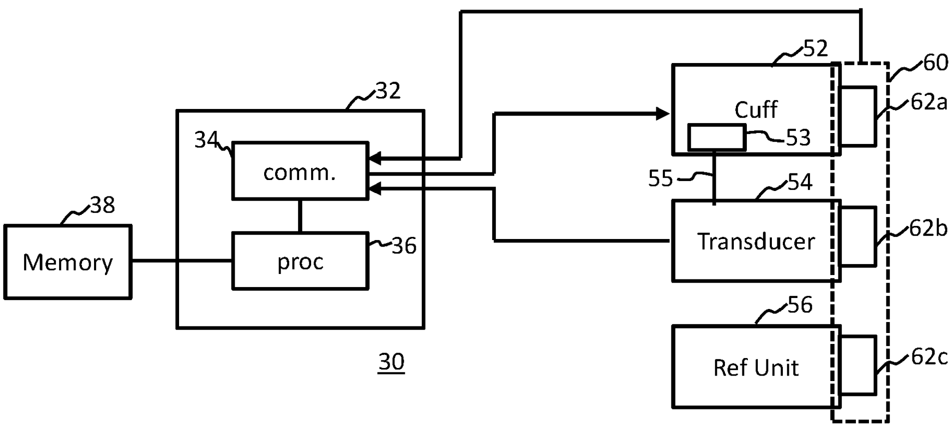


FIG. 4

EP 4 483 792 A1

## Description

FIELD OF THE INVENTION

The present invention relates to the field of non-invasive hemodynamic parameter measurement.

BACKGROUND OF THE INVENTION

The most common method for non-invasive blood pressure (NIBP) measurement is the use of an oscillometric blood pressure measurement cuff. The cuff comprises an inflatable bladder. The cuff wraps around the upper arm of a patient and is controlled to progress through an inflation and deflation cycle during which a pressure inside the bladder is sampled and a single set of values for systolic arterial pressure, mean arterial pressure and diastolic arterial pressure (and pulse pressure) are derived through processing of the pressure signal.

A variant approach to non-invasive blood pressure measurement has previously been proposed. This also uses a cuff which wraps around the body part of the patient. A cross-sectional illustration is shown in Fig. 1. The cuff 2 is shown wrapped around the arm 10 of a patient, and the brachial artery 8. The cuff 2 includes a pressure actuator 14, which optionally may be an inflatable bladder, as in the traditional NIBP cuff. In variance with the traditional NIBP cuff, the variant cuff also includes a separate tissue pressure sensor 4 arranged to measure a pressure between the body part 10 and the cuff. The tissue pressure sensor in some examples comprises a fluid-filled sensor pad, wherein a pressure in the pad varies as a function of pressure exerted on the pad. The pad may be fluidically coupled to a pressure transducer for read-out of a tissue pressure signal. When blood pulses in the artery 8, this results in a pressure wave 6 which can be detected by the tissue pressure sensor 4. The tissue pressure sensor 4 can be connected to the pressure transducer via a fluid-filled tube/line. The pressure transducer converts the pressure in the fluid into an electrical signal.

Also in variance with the traditional NIBP cuff, the variant cuff includes a hard shell 12 which extends around the body part and is disposed (radially) between the actuator 14 and the body part 10.

The pulse waves in the brachial artery 8 cause a change in the arm volume which causes a compression of the liquid in the sensor pad 4. The sensor pad is fluidly connected via a flexible tube filled with fluid to the pressure transducer which measures the pressure signal.

The pressure sensor pad 4 is enclosed by a hard shell 12 to enable a sufficient signal from the pressure sensor. In similarity with a standard NIBP cuff, the pressure actuator 14 may comprise a bladder which is inflatable with an air pump, to compress the arm tissue, so that the brachial artery 8 can be closed.

Due to the inclusion of the separate tissue pressure sensor 4 with hard shell 12 backing, this cuff can obtain a real time pulse signal for the patient, and, from this, multiple different hemodynamic parameters can be obtained, including stroke volume and cardiac output.

The shell structure 12 has an annular form, and, in the most common design, is formed from a single piece of material which is curled round to span a complete (closed) loop. Fig. 2 schematically shows an example shell structure 12 according to the state of the art. Fig. 2 (left) shows a cross-section across a plane normal to a longitudinal axis of the shell and parallel with tangential and radial axes of the shell. Fig. 2 (right) shows a cross-section across a plane parallel with a longitudinal axis of the shell.

The shell structure is for extending around the body part 10 and is for arrangement between the tissue pressure sensor and the pressure actuator during operation, the shell structure being discontinuous circumferentially so as to having at least one pair of tangentially overlapping edge surfaces 13a, 13b which are tangentially slidable relative to one another during operation.

In order to obtain accurate blood pressure measurements using conventional non-invasive inflatable blood pressure (NIBP) cuffs, it is important that the cuff is vertically aligned with the patient’s heart level. Patient motion and/or changes in patient posture have an impact on blood pressure measurements and consequently, on the reliability of the information obtained. This requirement is also true for the shell-backed cuff described above.

An additional requirement for the shell-backed cuff arises from the fluid-based tissue pressure sensor. As mentioned above, this comprises a fluid-filled pad coupled hydraulically to a pressure transducer unit via a fluid line. The fluid line for example is a fluid conduit in the form of a flexible hose or tube. In order to obtain accurate tissue pressure measurements with the tissue pressure sensor, the pressure transducer unit needs to be arranged level with the phlebostatic axis (PA) of the patient as well as the cuff being level with the phlebostatic axis. If the cuff or transducer unit is placed above the PA, erroneously low readings are obtained, whereas if the cuff or transducer is below the PA, erroneously high readings are obtained.

In clinical practice, maintaining alignment of the cuff and the transducer unit with the phlebostatic axis can be challenging. For example, even if the units begin at the correct levels, changes in patient position can bring them out of correct leveling. For example, the transducer unit may typically be attached to an exterior of the cuff. If the patient rotates from supine to lying sideways or vice versa, or if the transducer falls from its attached position or is relocated, then the

levelling is lost. In such situations, the transducer level is no longer aligned with the phlebostatic axis of the patient and thus measurement inaccuracy arises.

In such an eventuality, in the state of the art, manual corrective intervention of the operator or clinician is needed. In general, two alternative options are available.

A first option is physical re-positioning of the transducer unit so as to be bring it back to the correct level, i.e. to the height level of the phlebostatic axis. This may involve detaching or re-attaching the transducer unit. For example, in case the patient has been rotated, such that the transducer unit is no longer on the level of the phlebostatic axis, the transducer unit could be detached from the cuff and attached to other object (e.g., a dedicated holder), which is at the level of the phlebostatic axis. In another example, when the patient has not been moved, but the transducer unit has fallen from the cuff, the transducer unit can be simply reattached to the cuff.

A second option is application of a corrective algorithm to measurements to compensate for the height misalignment. Here, instead of physically bringing the transducer unit back to the level of the phlebostatic axis, the height displacement is measured by the operator and used as a correction term in measurement algorithms. In this case, the monitoring system typically comprises a user interface permitting the clinician to enter the correction term.

## SUMMARY OF THE INVENTION

It is the recognition of the inventors that the manual procedures mentioned above are sub-optimal since they interfere with a streamlined clinical workflow. Furthermore, these depend upon the clinician noticing the misalignment and thus impose the risk that incorrect levelling remains undetected.

It is the recognition of the inventors therefore that there would be advantage in providing a mechanism for automatically detecting and monitoring any height disparity between the transducer unit and/or the cuff with respect to the level of the phlebostatic axis. It would also be of benefit to provide a means for automatically compensating for any detected height disparity without having to manually re-level the system or perform any other manual transducer levelling procedures.

The invention is defined by the claims.

According to examples in accordance with an aspect of the invention, there is provided a system for use in blood pressure measurement. The system comprises a cuff for wrapping around a body part of a user, and wherein the cuff includes an actuator for use in applying a variable pressure to the body part by the cuff. The actuator may comprise an inflatable bladder in some embodiments. The system further comprises a tissue pressure sensor arranged for sensing a pressure between a surface of the body part and the cuff. The tissue pressure sensor comprises: a fluid-containing pad arranged so as to be in pressure-coupled relationship with the tissue of the body part when the cuff is wrapped around the body part, wherein changes in said pressure between a surface of the body part and the cuff are coupled to changes of fluid pressure in the pad. The tissue pressure sensor further comprises a pressure transducer unit fluidically coupled via a fluid line to the fluid pad and comprising readout electronics for transducing pressure exerted on the pad to an output tissue pressure signal (P(t)).

The system further comprises a height reference unit for attachment at a reference location on the body of the user (for example to a torso of the user, for example to the chest of the user).

The system further comprises a height sensing sub-system adapted to sense and monitor changes in:

a first relative height (H1), between the cuff and pressure transducer unit, and
a second relative height (H2) between the cuff and the reference unit.

The system preferably further comprises a controller adapted to compute a correction parameter ($\Gamma(t)$) for the tissue pressure signal (P(t)) based on changes in the first and second relative heights using a pre-determined function.

Height in this context means height in the direction of gravity. Relative height between two objects means a distance between those two objects in a height direction, i.e. a direction of gravity.

The pressure pad may be disposed between the actuator and the tissue surface.

Thus, it is proposed to monitor changes in a relative height (i.e. effectively a difference in absolute height) between: the cuff and a reference unit; and the transducer unit and the reference unit. The purpose of the hight reference unit is to provide a physical body whose height level can be used as a proxy marker for the height level of the patient phlebostatic axis. It is not essential that the reference unit be positioned exactly at the level of the phlebostatic axis. All that is needed is that the reference unit is attached or coupled to a part of the body whose motion is coupled with (follows) the motion of the phlebostatic axis. Thus, this allows for accounting for variations in the height of the phlebostatic axis itself when monitoring the changes in H1 and H2. The position changes of the reference unit thereby may act as a proxy for position changes in the phlebostatic axis.

By monitoring changes in H1 and H2 therefore, the system detects drift or shifts in the levelling of the cuff and the transducer unit relative to the phlebostatic axis. In practice, the measurement procedure may begin with the clinician

correctly levelling the cuff and the transducer unit, following which the monitoring and compensation method outlined above can be triggered, meaning that any deviations in levelling can be accounted for.

The height reference unit may include local independent means for monitoring height, or its height may be monitored by external means. Options will be discussed in greater detail in descriptions to follow.

The height reference unit may be body-mountable.

The height reference unit may for example be for mounting at a location of the heart or chest. It may be for mounting at the level of the phlebostatic axis but, as explained above, this is not a requirement for the system to work.

In some embodiments, the height reference unit may be mountable to the chest by a chest band for example.

In some embodiments, the height sensing sub-system is adapted to independently detect changes in height of each of the reference unit, the cuff and the pressure transducer unit, and to determine the changes in the first and second relative heights based thereon. This ensures that changes in height level of the phlebostatic axis itself, as reflected by changes in height of the reference unit, are taken into account.

In some embodiments, the height sensing sub-system is adapted to compute at each of a series of sampling points, t, spanning a monitoring period:

a net change ($\Delta$H1(t)) in the first relative height since a start of the monitoring period; and
a net change ($\Delta$H2(t)) in the second relative height since a start of the monitoring period.

By computing net change, there is provided a quasi real-time signal at each time point, t, indicative of the height disparity of each of the cuff and the transducer unit from correct levelling. This allows for a correction factor to be updated recurrently at each time point if necessary.

In some embodiments, the height sensing sub-system comprises a first motion sensor integrated in the cuff, a second motion sensor integrated in the pressure transducer unit and a third motion sensor integrated in the height reference unit. The height sensing sub-system may be adapted to monitor the changes in the first and second relative heights based on monitoring motion of each of the cuff, pressure transducer unit and height reference unit in the height direction. The height direction is gravitational vertical. Motion of the units, if monitored continuously or in real time, can be converted into a measure of net change in position since a starting time point.

In particular, in some embodiments, the controller is adapted to compute a net change in height, at time t of a monitoring period, of each of the cuff, pressure transducer unit and height reference unit based on aggregating a respective motion in the height direction of each of the cuff, pressure transducer unit and height reference unit between a start of the monitoring period and time t.

In some embodiments, each of the first, second and third motion sensors comprises a respective acceleration sensor (e.g. an accelerometer or an inertial measurement unit (IMU)) adapted to output a 3D acceleration signal, and wherein the controller is adapted to monitor motion in a height direction for each of the cuff, pressure transducer unit and height reference unit based on a component of the 3D acceleration signal of each respective IMU in a direction of gravity.

There are standard techniques for continuous resolving of the gravity component of a 3D accelerometer independent of orientation changes, e.g. tilt compensation.

The IMU may include at least a 3D accelerometer and may also include a gyroscope and/or magnetometer. The IMU may comprise only a 3D accelerometer.

In some embodiments, the controller is adapted to compute a net change in height, at time t of a monitoring period, of each of the cuff, pressure transducer unit and height reference unit based on double integration with respect to time of the component of the respective acceleration signal in the direction of gravity for each of the first, second and third IMU.

An alternative to using motion sensors to track position change is to provide a position tracking means which is capable of directly detecting and tracking changes in position of each of the cuff, the transducer unit and the reference unit. For example, optical or electromagnetic position tracking systems could be used. In such systems, a tracking sensor is typically integrated in each unit whose position is to be tracked and a central unit communicates optically or electromagnetically with these tracking sensors to monitor position.

In some embodiments, the controller is adapted to periodically compare a net change in the first relative height to a first threshold and the net change in the second relative height to a second threshold and control a user interface to generate user feedback responsive to exceeding the threshold.

In some embodiments, the controller is adapted to compute a correction of the tissue pressure signal based on the function:

$$P_c(t) = P_0 + \Gamma(t)$$

where $\Gamma(t) = \alpha \Delta H1(t) + \beta \Delta H2(t)$.

where $P_c$ is the corrected tissue pressure measurement, $P_0$ is the original tissue pressure measurement, $\Gamma(t)$ is the correction parameter, $\alpha$ is a constant proportional to a density of the fluid contained in the pad, $\Delta H1(t)$ is the net change in the first relative height at time *t*, $\beta$ is a constant proportional to the density of blood, and $\Delta H2(t)$ is the net change in the second relative height at time *t.*

The constants $\alpha$ and $\beta$ have units of pressure per unit distance, for example mmHg/cm. The values of $\alpha$ and $\beta$ may be set at values determined according to the hydrostatic equation, $\Delta P = \rho * g * h$, where $\Delta P$ is the pressure difference, $\rho$ is the density of the relevant fluid (either blood or hydraulic fluid), g is the acceleration due to gravity, and h is the height difference.

For example, the density of blood is $\rho$ = 1060kg/m$^3$. Therefore, a value of $\beta$ in units of mmHg/cm may be determined as $\beta$ = 1060kg/m$^3$ * 9.81 * 0.01 = 103.986 Palcm = 0.78 mmHg/cm.

For the pressure transducer, the value of $\alpha$ may be determined in dependence upon the density of the hydraulic fluid. For example, an expression for the value of $\alpha$ in units of mmHg/cm may be determined as: $\alpha = (\rho * 9.81 * 0.01) / 133.322 = \rho * 7.358 \times 10^{-4}$, where $\rho$ is the density of the hydraulic fluid.

In some embodiments, $\alpha$ = 0.69 mmHg / cm height difference.

This applies for an example hydraulic fluid of silicon oil, having a density of between 760 and 1070 kg/cm$^3$, e.g. approximately 940 kg/cm$^3$.

In some embodiments, $\beta$ = 0.78 mmHg / cm height difference, assuming a density of blood of 1060 kg/m$^3$.

In some embodiments, the pressure transducer unit, cuff and/or reference unit comprises an IMU adapted to output an acceleration signal and wherein the controller is further adapted to detect motion events of the pressure transducer unit, cuff and/or reference unit using the acceleration signal output from the IMU. The controller may be adapted to generate user feedback through control of a user interface responsive to the detection. Additionally or alternatively, the same motion event detection and possible feedback may be performed using an IMU comprised by the cuff and/or the height reference unit.

In some embodiments, the controller is further adapted to determine a severity of each detected motion event based on a feature of the acceleration signal corresponding to the event. In some embodiments, the controller is adapted to classify a measurement quality of a blood pressure measurement obtained over a time period which coincided with the motion event. The quality classification may be determined based on a pre-determined mapping between motion event severity and measurement quality.

The feature of the acceleration signal may for example be a peak acceleration value during the motion event.

In some embodiments, instead of using motion tracking to monitor height changes, the height sensing subsystem may include a fluid line (conduit) connected between the height reference unit and the cuff and wherein the change in the relative height between the reference unit and the cuff is computed based on a change in a pressure differential between the reference unit and the cuff.

The height sensing subsystem may additionally or alternatively include a secondary fluid line (conduit) connected between the pressure transducer unit and the cuff and wherein the change in the relative height between the pressure transducer unit and the cuff is computed based on a change in a pressure differential between the pressure transducer unit and the cuff.

In some embodiments, the height sensing subsystem further comprises at least one air pressure sensor coupled to at least one of the cuff, the pressure transducer unit and the reference unit.

In some embodiments, the system may comprise a base unit, wherein the base unit houses the controller. The base unit may comprise a user interface including a display device. The base unit may include a hemodynamic measurement module for computing one or more hemodynamic measurements based on the tissue pressure signal.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an example apparatus suitable for use as part of embodiments of the present invention;
Fig. 2 shows a schematic representation of a shell portion of the apparatus of Fig. 1;
Fig. 3 schematically illustrates the positioning of the phlebostatic axis;
Fig. 4 is a block diagram of an example system in accordance with one or more embodiments of the invention;
Fig. 5 illustrates a schematic plan view of placement of the height reference unit, the cuff and the pressure transducer unit;
Fig. 6 illustrates a schematic side elevation view of placement of the height reference unit, the cuff and the pressure

transducer unit;
Fig. 7 illustrates a cross-section view normal to a long axis of the patient body, showing placement of the height reference unit, the cuff and the pressure transducer unit when all three are levelled;
Fig. 8 and Fig. 9 each illustrate an example disruption of the alignment of Fig. 7 caused by patient motion;
Fig. 10 illustrates the parameters H1, H2;
Fig. 11 illustrates monitoring a change in H1 and H2 between a starting time, to, and a later time point $t_n$; and
Fig. 12 illustrates determining a net height change of each of the cuff, reference unit and pressure transducer unit, at time $t=t_n$, based on integration of an acceleration signal from each unit.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for use in non-invasive hemodynamic parameter measurement which comprises a cuff having a pressure actuator and a tissue pressure sensor, where the tissue pressure sensor comprises a fluid pad hydraulically connected to a fluid pressure transducer unit. Provided is a height monitoring means for tracking a change in a height of the transducer unit relative to the cuff and the cuff relative to a heart level of the patient, wherein the heart level is indicated by proxy using a height reference unit mounted to the patient's torso. Changes in these relative heights are used to compute a correction parameter for the tissue pressure signal. In some embodiments, changes in height of each of the cuff, transducer unit and height reference unit may be independently tracked. In some embodiments, this is done by motion tracking of each unit and aggregating net motion to determine position change.

In order to obtain accurate readings of hemodynamic measurements and to prevent pressure offset, it is preferable that the cuff and the transducer unit are located approximately at the level of the phlebostatic axis. This levelling is preferably maintained throughout the whole procedure.

Fig. 3 schematically illustrates the positioning of the phlebostatic axis 58 relative to a patient 70. Fig. 3 shows a side view of the patient, with the torso 74 and right arm 72 shown. The phlebostatic axis is located at the level of the right atrium, approximately at the fourth intercostal space (the space between the fourth and fifth ribs) in the mid-axillary line (a vertical line running down the side of the torso roughly halfway between the anterior and posterior axillary folds).

In clinical practice, maintaining alignment of the cuff and the transducer unit with the phlebostatic axis can be challenging. For example, even if the units begin at the correct levels, changes in patient position can bring them out of correct leveling. For example, the transducer unit may typically be attached to an exterior of the cuff. If the patient rotates from supine to lying sideways or vice versa, or if the transducer falls from its attached position or is relocated, then the levelling is lost. In such situations, the transducer level is no longer aligned with the phlebostatic axis of the patient and thus measurement inaccuracy arises.

In such an eventuality, in the state of the art, manual corrective intervention of the operator or clinician is needed. These manual corrective actions require additional steps in the clinical workflow which is time consuming and may result in inaccurate measurements. In addition, there is a risk that incorrect levelling may go undetected.

Embodiments of the present invention aim to overcome the shortcomings of the current approach by providing a system and method that automatically detects, determines and corrects the relative height difference of the patient's phlebostatic axis and the cuff and between the cuff and the transducer unit, avoiding manual transducer levelling procedures and ensuring accurate measurements.

In some embodiments, there is provided an approach for automatic correction of relative height changes of the cuff with regard to the phlebostatic axis of the patient and the transducer unit relative to the cuff.

In one set of embodiments, height and/or motion monitoring means are used together with a processing unit to detect and correct the levelling.

Fig. 4 outlines in block diagram form an example system 30 in accordance with one or more embodiments. The system is for use in non-invasive blood pressure and/or hemodynamic parameter measurement.

The system 30 comprises a cuff 52 for wrapping around a body part of a user, and wherein the cuff includes an actuator for use in applying variable pressure applied to the body part by the cuff. The actuator is not shown in Fig. 4 but is illustrated in Fig. 1. The actuator may comprise an inflatable bladder in some embodiments.

The system further includes a tissue pressure sensor arranged for sensing a pressure between a surface of the body part and the cuff.

The tissue pressure sensor comprises: a fluid-containing pad 53 arranged so as to be in pressure-coupled relationship with the tissue of the body part when the cuff is wrapped around the body part, and wherein changes in said pressure between a surface of the body part and the cuff are coupled to changes of fluid pressure in the pad.

The tissue pressure sensor further comprises a pressure transducer unit 54 fluidically coupled via a fluid line 55 to the fluid pad 53 and comprising readout electronics for transducing pressure exerted on the pad to an output tissue pressure signal (P(t)).

The system further includes a height reference unit 56 for attachment at a reference location on the body of the user.

The system further includes a height sensing sub-system 60 adapted to sense and monitor changes in:

a first relative height (H1), between the cuff 52 and the pressure transducer unit 54, and
a second relative height (H2) between the cuff 52 and the height reference unit 56.

The system further comprises a controller 32 adapted to compute a correction parameter ($\Gamma(t)$) for the tissue pressure signal (P(t)) based on changes in the first and second relative heights using a pre-determined function.

In the illustrated example of Fig. 4, the height sensing subsystem 60 includes a tracking or sensing element 62a, 62b, 62c incorporated in each of the cuff 52, transducer unit 54 and height reference unit 56. As will be explained below, in some embodiments, this may take the form of a respective acceleration sensor or IMU carried by each unit. The height sensing subsystem may communicate with the controller. The height sensing subsystem may include one or more processors which are arranged communicatively coupled with the controller 32. In some embodiments, processing or computation functions of the height sensing subsystem may be performed by the controller itself.

Height in this context means height in the direction of gravity.

Relative height between two objects means a distance between those two objects in a height direction, i.e. a direction of gravity.

The pressure pad 53 may be disposed between the actuator and the tissue surface.

The controller 32 may comprise one or more processors 36, as illustrated in Fig. 4. In the illustrated example, the processing device further comprises an input/output 34 or communication interface. This may receive signals or data from the height sensing subsystem 60 for example. It may receive signals from the tissue pressure transducer 54 in some examples.

The system 30 may further comprise a memory 38 for storing computer program code (i.e. computer-executable code) which is configured for causing the one or more processors 36 of the processing unit 32 to perform computation or control steps in accordance with any of the embodiments described in this document.

With regards to the height reference unit 56, a primary purpose of this unit is to provide a physical body whose height level can be used as a proxy marker for the height level of the patient phlebostatic axis. It is not essential that the reference unit be positioned exactly at the level of the phlebostatic axis. All that is needed is that the reference unit is attached or coupled to a part of the body whose motion is coupled with (follows) the motion of the phlebostatic axis. Thus, this allows for accounting for variations in the height of the phlebostatic axis itself when monitoring the changes in H1 and H2. The position changes of the reference unit thereby may act as a proxy for position changed in the phlebostatic axis.

The reference unit 56 may contain components for autonomously tracking its own height, and/or a separate system may be provided for tracking the height from an external position, e.g. an optical and/or electromagnetic tracking system. The reference unit 56 may include sensing elements for interfacing or communicating with such a system.

A preferred approach employs an inertial measurement unit (IMU), e.g., comprising a 3D accelerometer and optionally comprising gyroscope sensors. The IMU provides an indication of acceleration and may provide an indication of orientation change. As will be explained below, by double integration of this acceleration signal, a measure of a change in height over time can be determined. This therefore provides a means to detect and measure position and possible changes of position of a patient's phlebostatic axis during hemodynamic monitoring. Optionally, an additional air pressure sensor is provided.

The reference unit 56 enables to determine changes of its position and particularly measurement of height changes. In some embodiments, the reference unit 56 may be directly attached to the patient's chest (close to heart location) or integrated into a body-worn device on the patient.

In some embodiments, the height reference unit may additionally incorporate one or more physiological parameter sensors, such as a heart rate sensor and/or ECG sensor. This enables it to advantageously perform two functions simultaneously.

The reference unit 56 may comprise a microprocessor with a wireless (or wired) transceiver able to communicate with the controller 32 and/or to the transducer unit 54.

To detect and measure changes of position of the transducer unit 54, in some embodiments, this may also comprise an inertial measurement unit (IMU) integrated therein, e.g., comprising a 3D accelerometer and optionally gyroscope sensors. The IMU provides an indication of acceleration and optionally orientation change. This enables

determination of changes of the transducer unit position and particularly measurement of height changes.

Optionally, an additional air pressure sensor is integrated into the transducer unit 54.

The transducer unit 54 is communicatively connected to the controller 32 with either wired or wireless connection. The transducer unit may comprise a local microprocessor.

With regards to the cuff comprising the actuator, the cuff described previously with reference to Fig. 1 and Fig. 2 may be used. Details will not be repeated for purposes of brevity.

Fig. 5 schematically illustrates an example placement of the height reference unit 56, cuff 52 and pressure transducer unit 54 during operation. As illustrated, the height reference unit 56 may be mounted to a chest of the patient, the cuff 52 mounted to an upper arm, and the transducer unit 54 coupled to the cuff. These positions are not essential and represent just one example.

In some embodiments, the system comprises a base unit, wherein the base unit houses the controller. The base unit may comprise a user interface including a display device. The base unit may include a hemodynamic measurement module for computing one or more hemodynamic measurements based on the tissue pressure signal.

The base unit may take the form of a patient monitor unit. The patient monitor may be adapted to automatically obtain and process the information from the height sensing subsystem 60. It also receives a tissue pressure signal from the tissue pressure sensor transducer 54 for use in computing hemodynamic parameters. The base unit may also control operation of the cuff during a measurement cycle, i.e. controlling the applied pressure cycle over the measurement. For example this may comprise controlling an inflation/deflation cycle of an inflatable bladder.

The correction parameter determined by the controller 32 may be used by the base unit in computing hemodynamic parameters. In some embodiments, computation of the hemodynamic parameters and determination of the correction factor may be performed by the same controller 32.

As explained above, for accurate measurement, the cuff and transducer unit should be aligned level with the phlebostatic axis. If the cuff is raised or lowered relative to the phlebostatic axis, error arises in pressure measurements because of the additional distance travelled by the blood in the direction of gravity to the position of the cuff, this being against gravity if the cuff is raised and with gravity if the cuff is lowered. Likewise, the transducer unit should be level with the pressure pad or else gravitational effects add an additional hydrostatic offset (positive or negative) to the hydraulic fluid pressure.

In standard clinical practice, for simplicity, it is assumed that, regardless of the position of the cuff, if the transducer unit is at the same level as the phlebostatic axis, then the measurement is sufficiently accurate. This is because, to first approximation, any additional hydrostatic pressure arising at the cuff due to vertical offset of the cuff from the heart is countered by the equal and opposite vertical offset of the transducer unit relative to the cuff. Thus the pressure measured at the transducer unit is approximately accurate.

It is the recognition of inventors of the present invention that an improved accuracy can be obtained if changes in the height disparity between the cuff and the transducer unit and the height disparity between the cuff and the phlebostatic axis are separately monitored. This then allows for taking into account the different fluid densities of blood versus hydraulic fluid, and thus a separate hydrostatic correction can be applied for each and then summed together.

As discussed previously, the problem being addressed is the loss of vertical alignment between the cuff, transducer unit and phlebostatic axis. The alignment can become lost upon certain changes of position of the patient or due to action by the clinician.

This is illustrated in Fig. 6-9. Fig. 6 and Fig 7 schematically shows the ideal positioning of the cuff 52, the transducer unit 54 and the height reference unit 56, all approximately aligned with a phlebostatic axis 58 of the patient. However, during measurement, it can occur that a patient moves their pose so that one or more of the cuff and the pressure transducer ceases being level with the phlebostatic axis. Fig. 8 for instance illustrates an example where the patient stays lying in a supine position but lifts up the arm carrying the cuff and the transducer unit. Accordingly, the cuff and the transducer unit are vertically elevated relative to the phlebostatic axis. Fig. 9 illustrates a further example in which the patient turns to their side. Again, this results in the cuff 52 and the transducer unit 54 becoming elevated relative to the phlebostatic axis.

Thus, the proposed concept is to track height change of the cuff 52 and the transducer unit 54 over time.

To implement this, the height reference unit 56 is introduced for mounting to the chest of the patient, and wherein tracking a change in the height of the reference unit gives a proxy measure for the change in the height level of the phlebostatic axis.

Aside from the cuff and the transducer unit, it can also occur that the level of the patient's chest independently changes, e.g. if the patient sits up, or otherwise changes the pose of their torso. Thus, it is the recognition of the inventors that maximal reliability of the tracking method is ensured if height changes in each of the cuff 52, the transducer unit 54 and the reference unit 56 are each independently tracked.

To track the heights, according to at least one set of embodiments, each unit is provided with a motion sensor such as an inertial measurement unit and an example implementation in accordance with this tracking approach is now discussed in more detail.

For the purposes of compensating the hemodynamic measurements, the relevant parameters are the height difference between the cuff 52 and the transducer unit 54 and the height difference between the cuff 52 and the phlebostatic axis 58. The height of the phlebostatic axis is reflected by the height of the reference unit 56.

Thus the key parameters which are sought to be tracked are the relative height (or absolute height difference) between the cuff 52 and the pressure transducer unit 54 (H1); and the relative height (or absolute height difference) between the cuff 52 and the reference unit 56 (H2).

These parameters are illustrated visually in Fig. 10. For simplicity of reference, all of the units are shown in block diagram form. This is purely schematic.

In operation, the cuff 52, transducer unit 54 and height reference unit 56 may be initially applied to the patient, such that at least the cuff 52 and transducer unit are approximately level with the phlebostatic axis. Typically, this is the case when the patient is in supine position (e.g., during anaesthesia induction at surgery preparation) and the cuff is attached to the upper arm with the transducer unit being attached to the cuff. The cuff may carry a graphical marker indicating the preferred positioning for attachment of the transducer unit. The height reference unit may be mounted to the body of the patient, preferably close to the heart, and therefore close to the phlebostatic axis. It should be mounted to a part of the body whose movement is coupled to the movement of the phlebostatic axis, such that changes in height of the phlebostatic axis are coupled to changes in height of the reference unit 56.

A value of the relative height, H1, between the cuff and pressure transducer is reset to zero. A value of the relative height, H2, between the cuff and the reference unit 56 is reset to zero. This initial calibration may be achieved for example by the operator pressing a calibration button on a provided user interface.

It is noted that the system in accordance with the present invention allows for the possibility of some initial height offset between the cuff 52 and transducer unit 54 and/or the cuff 52 and the reference unit 56. This initial offset may simply be measured manually by an operator, input to the controller or the height subsystem via a user interface, and wherein these initial values are used in the computation of the correction parameter. In other words, a user interface may be provided and wherein the height sensing subsystem is adapted to receive from the user interface:

an initial value, H1_0, for the relative height between the cuff and pressure transducer unit, and
an initial value, H2_0, for the relative height between the cuff and the reference unit.

Changes in H1 and H2 may be determined relative to the initial values.

However, such input of initial values is not essential. Instead, as mentioned above, the system may simply take these values to be zero. In some embodiments, the user has an option to input starting values H1_0 and H2_0 or to simply press a 'reset' or 'calibration' button which sets both of these values to zero.

After the initial calibration, the system continuously tracks the relative height displacement of the transducer unit 54 relative to the cuff 52 and the cuff 52 relative to the height reference unit 56. As will be explained below, this may be achieved by continuously calculating height changes of each of the cuff 52, transducer unit 54 and the height reference unit 56. Height changes can be calculated based on the raw sensor data from the different sensors in the sensing units, for example by double-integration of acceleration data of a 3D accelerometer in the direction of gravity. In some embodiments, more accurate height information can be obtained by fusing the data from more than one type of sensor, for example from a 3D accelerometer, a gyroscope, and/or an air pressure sensor.

From the height changes of the cuff 52, transducer unit 54 and reference unit 56, changes in the relative heights H1, H2 can be calculated.

Note, that the calculation of height changes of each of the cuff 52, transducer unit 54 and reference unit 56 can be done either locally at the sensor units 62a, 62b, 62c of the relevant units, or may be done centrally by a dedicated processor provided to the height sensing subsystem 60, or may be done centrally by the controller 32.

It needs to be noted this approach assumes that relative height difference, H2, between the cuff 52 and the and the reference unit 56, after initial calibration, represents the relative height displacement of the cuff with respect to the phlebostatic axis. However, in practice the reference unit is not exactly on the level of the phlebostatic axis, therefore this assumption represents an approximation only.

The methods for tracking the height changes and compensating therefor will now be discussed in more detail, with reference to Fig. 11 and Fig. 12.

As briefly mentioned above, in a preferred set of embodiments, the height sensing sub-system 60 is adapted to independently detect changes in: height (Hr) of the reference unit, height (He) of the cuff, and height (Ht) of the pressure transducer unit, and to determine the changes in the first (H1) and second (H2) relative heights based thereon.

In a preferred set of embodiments, the height sensing sub-system may be adapted to compute at each of a series of sampling points, t, spanning a monitoring period:

a net change ($\Delta H1(t)$) in the first relative height since a start of the monitoring period ($t=t_0$); and
a net change ($\Delta H2(t)$) in the second relative height since a start of the monitoring period.

This is illustrated schematically in Fig. 11.

Fig. 11 (top) shows an illustrative set of starting heights for each of the reference unit 56, the cuff 52 and the transducer unit 54 at starting time t=to. For purposes of simplicity, in this example the starting height of the reference unit (Hr_0), the starting height of the cuff (Hc_0) and the starting height of the pressure transducer unit (Ht_0), at time t=to, are taken to all be the same, i.e. the cuff 52 is level with the transducer unit 54 and the cuff 52 is level with the height reference unit 56. However, the proposed calibration method does not depend on this.

In practice, the height reference unit may be positioned on the chest such that the height (Hr) of the height reference unit approximately reflects a height of the phlebostatic axis.

Fig. 11 (bottom) shows an illustrative set of heights of the three units at later time $t=t_n$. In the illustrated example, at $t=t_n$, the height of the reference unit (Hr_n) has declined, the height of the cuff (Hc_n) has remained the same, and the height of the pressure transducer unit (Ht_n) has risen. This might arise for example if the patient has elevated their arm at an angle, whereby the cuff is raised above the chest, and the transducer unit, e.g. attached to the cuff toward a distal end, becomes raised relative to a center point of the cuff. It will be appreciated that this is purely an example illustration, and the method has general applicability for any values of Hr_n, Hc_n, Ht_n at the later time point, $t_n$. The time point $t_n$ is intended to represent any general later time point during the measurement session.

At each time point, $t_n$, the height sensing subsystem may compute a net change ($\Delta H1(t_n)$) in the first relative height since the start of the monitoring period (t=to); and a net change ($\Delta H2(t_n)$) in the second relative height since a start of the monitoring period.

For example, these values may be computed as follows:

$$\Delta H2 = \Delta Hc - \Delta Hr = [Hc_n - Hc_0] - [Hr_n - Hr_0]$$

$$\Delta H1 = \Delta Hc - \Delta Ht = [Hc_n - Hc_0] - [Ht_n - Ht_0]$$

where

$\Delta H2$ is the net change in the value of H2 since the start of the measurement session at $t=t_0$;
$\Delta H1$ is the net change in the value of H1 since the start of the measurement session at $t=t_0$;
$\Delta Hc$ is the net change in the height He of the cuff 52 since the start of the measurement session at $t=t_0$;
$\Delta Hr$ is the net change in the height Hr of the reference unit 56 since the start of the measurement session at $t=t_0$;
$\Delta Ht$ is the net change in the height Ht of the transducer unit 54 since the start of the measurement session at $t=t_0$;
Hc_n is the value of the height He of the cuff 52 at time $t=t_n$;
Hr_n is the value of the height Hr of the reference unit 56 at time $t=t_n$;
Ht_n is the value of the height Ht of the transducer unit 54 at time $t=t_n$;
Hc_0 is the starting value of the height He of the cuff 52 at time $t=t_0$;
Hr_0 is the starting value of the height Hr of the reference unit 56 at time $t=t_0$;
Ht_0 is the starting value of the height Ht of the transducer unit 54 at time t=to.

In accordance with a preferred set of embodiments, it is proposed to avoid a need to continuously track absolute values of He, Hr and Ht, but to instead simply track values of $\Delta Hc$, $\Delta Hr$, $\Delta Ht$, such that changes in H1 and H2 as a function of time can simply be computed as

$$\Delta H2(t) = \Delta Hc(t) - \Delta Hr(t)$$

$$\Delta H1(t) = \Delta Hc(t) - \Delta Ht(t).$$

This approach will now be discussed.

The height sensing sub-system 60 comprises a first motion sensor 62a integrated in the cuff 52, a second motion sensor 62b integrated in the pressure transducer unit 54 and a third motion sensor 62c integrated in the height reference unit 56. The height sensing sub-system may be adapted to monitor the changes in the first (H1) and second (H2) relative heights based on monitoring motion of each of the cuff, pressure transducer unit and height reference unit in the height direction. The height direction is a gravitational vertical.

Based on this, the controller 32 or the height sensing subsystem 60 may be adapted to compute a net change in height, at time t of a monitoring period, of each of the cuff 52, pressure transducer unit 54 and height reference unit 56 based on aggregating a respective motion in the height direction of each of the cuff, pressure transducer unit and height reference unit between a start of the monitoring period and time t.

For example in a preferred set of embodiments, each of the first 62a, second 62b and third 62c motion sensors comprises a respective IMU adapted to output a 3D acceleration signal, and wherein the controller is adapted to monitor motion in a height direction for each of the cuff, pressure transducer unit and height reference unit based on a component of the 3D acceleration signal of each respective IMU in a direction of gravity.
There are standard techniques for continuous resolving of the gravity component of a 3D accelerometer independent of orientation changes, e.g. tilt compensation.
The IMU may include at least a 3D accelerometer and may also include a gyroscope and/or magnetometer.
The controller may compute a net change in height, at time t of a monitoring period, of each of the cuff 52, pressure transducer unit 54 and height reference unit 56 based on double integration with respect to time of the component of the respective acceleration signal in the direction of gravity for each of the first, second and third IMU.
This is illustrated schematically in Fig. 12.
Fig. 12 schematically illustrates an example extracted acceleration component in the direction of gravity for each of the reference unit (a_r), the cuff (a_c) and the pressure transducer unit (a_t).
The dashed vertical line illustrates an example time point $t_n$. Computation of the net change in height of each unit may be achieved by double integration with respect to time of the component of the respective acceleration signal in the direction of gravity for each of the three units.
These may be computed as follows:

$$\Delta Hr(t_n) = \iint_{t=t0}^{t=tn} a_r dt^2$$

$$\Delta Hc(t_n) = \iint_{t=t0}^{t=tn} a_c dt^2$$

$$\Delta Ht(t_n) = \iint_{t=t0}^{t=tn} a_t dt^2$$

where $\Delta Hr(t_n)$ is the net change in height of the reference unit 56 at time point $t=t_n$ since a starting time $t=t_0$;
$\Delta Hc(t_n)$ is the net change in height of the cuff 52 at time point $t=t_n$ since a starting time $t=t_0$;
$\Delta Ht(t_n)$ is the net change in height of the transducer unit 54 at time point $t=t_n$ since a starting time $t=t_0$.

From these values, the changes in H1, H2 can then be evaluated at each time point $t_n$ by:

$$\Delta H2(t_n) = \Delta Hc(t_n) - \Delta Hr(t_n)$$

$$\Delta H1(t_n) = \Delta Hc(t_n) - \Delta Ht(t_n).$$

Different actions can be taken in response to the obtained values for change in H1 and change in H2.
In some embodiments, the controller is adapted to periodically compare a net change in the first relative height to a first threshold and the net change in the second relative height to a second threshold and control a user interface to generate user feedback responsive to exceeding the threshold. By way of illustration, a sample threshold might be between 1-3 cm. The user interface may be a user interface of a base unit or patient monitor unit as discussed previously. The provided feedback may include a notification (e.g. graphical or audio) that the cuff or transducer unit (as appropriate) has drifted from alignment. The feedback may include advice or guidance (e.g. graphical or audio) to correct the levelling, e.g. to bring the cuff and/or transducer unit back to the level of the phlebostatic axis.
Additionally or alternatively, the change in H1 and H2 may be used to automatically correct the tissue pressure signal output from the pressure transducer unit.
The controller 32 may be adapted to compute a correction of the tissue pressure signal, P, based on the function:

$$P_c(t) = P_0(t) + \Gamma(t)$$

where $\Gamma(t) = \alpha\Delta H1(t) + \beta\Delta H2(t)$

where

$P_c(t)$ is the corrected tissue pressure measurement at time $t$;
$P_0(t)$ is the original (uncorrected) tissue pressure measurement (e.g. as received directly from the tissue pressure sensor) at time $t$;
$\Gamma$(t) is the correction parameter at time $t$;
$\alpha$ is a constant proportional to a density of the fluid contained in the pad;
$\Delta$H1(t) is the net change in the first relative height at time $t$;
$\beta$ is a constant proportional to the density of blood; and
$\Delta$H2(t) is the net change in the second relative height at time $t$.

The constants $\alpha$ and $\beta$ have units of pressure per unit distance, for example mmHg/cm. The values of $\alpha$ and $\beta$ may be set at values determined according to the hydrostatic equation, $\Delta P = \rho * g * h$, where $\Delta P$ is the pressure difference, $\rho$ is the density of the relevant fluid (either blood or hydraulic fluid), g is the acceleration due to gravity, and h is the height difference.

For example, the density of blood is $\rho = 1060 kg/m^3$. Therefore, a value of $\beta$ in units of mmHg/cm may be determined as $\beta = 1060 kg/m^3 * 9.81 * 0.01 = 103.986$ Palcm = 0.78 mmHg/cm.

For the pressure transducer, the value of $\alpha$ may be determined in dependence upon the density of the hydraulic fluid. For example, an expression for the value of $\alpha$ in units of mmHg/cm may be determined as: $\alpha = (\rho * 9.81 * 0.01) / 133.322 = \rho * 7.358 \times 10^{-4}$, where $\rho$ is the density of the hydraulic fluid. For example, in some embodiments, $\alpha = 0.69$ mmHg / cm height difference. This applies for an example hydraulic fluid of silicon oil, having a density of approximately 940 $kg/cm^3$.

In some embodiments, the motion sensors integrated in each of the cuff, transducer unit and height reference unit are used to detect motion of one or more of the components of the system. For example, the IMU in the transducer unit 54, the cuff 52 and/or the height reference unit 56 may be used to detect motion events. Those motion events might be caused for example by the operator or other clinicians unknowingly touching or hitting the cuff or the fluid line between the sensor pad and the transducer. This might occur for instance during transporting of the patient. Such motion events can result in motion artefacts in the tissue pressure signal, disrupting the calculation of blood pressure parameters and other hemodynamic parameters.

In some embodiments, the controller 32 may be adapted to generate user feedback through control of a user interface responsive to the detection of any motion event.

In some embodiments, the controller 32 is further adapted to determine a severity of each detected motion event based on a feature of the acceleration signal corresponding to the event. The feature of the acceleration signal may for example be a peak acceleration value during the motion event. The controller is adapted to classify a measurement quality of a blood pressure measurement obtained over a time period which coincides with the motion event. The quality classification is determined based on a pre-determined mapping between motion event severity and measurement quality.

For example, if motion events of severity exceeding a threshold are detected during a cuff measurement then the controller 32 may cause the cuff measurement to be aborted, and/or feedback may be generated for an operator or clinician, indicating that the measurement has been affected by motion artefacts.

By way of example, the following might be classified as severe events: at least one motion event with acceleration > 1 g (with 1 g ~ 9.8 $m/s^2$); three motion events with acceleration > 0.4 g; or one or more motion events with acceleration > 0.1 g and duration > 1 sec.

In some embodiments, instead of using motion tracking to monitor height changes, the height sensing subsystem may include a fluid line (conduit) connected between the height reference unit and the cuff and wherein the change in the relative height between the reference unit and the cuff is computed based on a change in a pressure differential between the reference unit and the cuff.

The height sensing subsystem may additionally or alternatively include a secondary fluid line (conduit) connected between the pressure transducer unit and the cuff and wherein the change in the relative height between the pressure transducer unit and the cuff is computed based on a change in a pressure differential between the pressure transducer unit and the cuff.

For example, in some embodiments, the cuff 52 and the height reference unit 56 may be connected by means of a fluid filled hose (e.g., water or a silicon oil). The cuff may contain another pressure transducer that transduces the pressure difference in the fluid hose from the reference unit 56 to the cuff 52 into an electrical signal. This approach has the drawback

of a ‘wired connection’ between the cuff and the reference unit. However, the advantage is that the height difference between the cuff and the reference unit is measured directly and therefore with high accuracy and reliability. The same can be done between the cuff 52 and the transducer unit 54 using a secondary dedicated fluid line for height monitoring purposes.

In some embodiments, the height monitoring means further comprises at least one air pressure sensor coupled to at least one of the cuff, the pressure transducer unit and the reference unit. This can be used to supplement or replace the height monitoring information derived using the motion sensors discussed above.

Embodiments of the invention described above comprise a controller. The controller unit may comprise one or more processors. It may be located in a single containing device, structure or unit, or it may be distributed between a plurality of different devices, structures or units. Reference therefore to the controller being adapted or configured to perform a particular step or task may correspond to that step or task being performed by any one or more of a plurality of controller components, either alone or in combination. The skilled person will understand how such a distributed processing device can be implemented. The controller may include a communication module or input/output for receiving data and outputting data to further components.

The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

**1.** A system (30) for use in blood pressure measurement comprising:

a cuff (52) for wrapping around a body part of a user, and wherein the cuff includes an actuator for use in applying a variable pressure to the body part by the cuff,

a tissue pressure sensor arranged for sensing a pressure between a surface of the body part and the cuff, wherein the tissue pressure sensor comprises:

a fluid-containing pad (53) arranged so as to be in pressure-coupled relationship with the tissue of the body part when the cuff is wrapped around the body part, wherein changes in said pressure between a surface of the body part and the cuff are coupled to changes of fluid pressure in the pad; and

a pressure transducer unit (54) fluidically coupled via a fluid line to the fluid pad and comprising readout electronics for transducing pressure exerted on the pad to an output tissue pressure signal (P(t));

a height reference unit (56) for attachment at a reference location on the body of the user;

a height sensing sub-system (60) adapted to sense and monitor changes in:

a first relative height (H1), between the cuff and pressure transducer unit, and
a second relative height (H2) between the cuff and the reference unit; and

a controller (32) adapted to compute a correction parameter ($\Gamma(t)$) for the tissue pressure signal (P(t)) based on changes in the first and second relative heights using a pre-determined function.

2. The system of claim 1, wherein the height reference unit (56) is body-mountable.

3. The system of claim 1 or 2, wherein the height sensing sub-system is adapted to independently detect changes in height of each of the reference unit, the cuff and the pressure transducer unit, and to determine the changes in the first and second relative heights based thereon.

4. The system of any of claims 1-3, wherein the height sensing sub-system is adapted to compute at each of a series of sampling points, t, spanning a monitoring period:

a net change ($\Delta H1(t)$) in the first relative height since a start of the monitoring period; and
a net change ($\Delta H2(t)$) in the second relative height since a start of the monitoring period.

5. The system of claim 3 or 4,

wherein the height sensing sub-system comprises a first motion sensor integrated in the cuff, a second motion sensor integrated in the pressure transducer unit and a third motion sensor integrated in the height reference unit, wherein the height sensing sub-system is adapted to monitor the changes in the first and second relative heights based on monitoring motion of each of the cuff, pressure transducer unit and height reference unit in the height direction.

6. The system of claim 5, wherein the controller is adapted to compute a net change in height, at time t of a monitoring period, of each of the cuff, pressure transducer unit and height reference unit based on aggregating a respective motion in the height direction of each of the cuff, pressure transducer unit and height reference unit between a start of the monitoring period and time t.

7. The system of claim 5 or 6,
wherein each of the first, second and third motion sensors comprises a respective inertial measurement unit adapted to output a 3D acceleration signal, and wherein the controller is adapted to monitor motion in a height direction for each of the cuff, pressure transducer unit and height reference unit based on a component of the 3D acceleration signal of each respective inertial measurement unit in a direction of gravity.

8. The system of claim 7, wherein the controller is adapted to compute a net change in height, at time t of a monitoring period, of each of the cuff, pressure transducer unit and height reference unit based on double integration with respect to time of the component of the respective acceleration signal in the direction of gravity for each of the first, second and third inertial measurement unit.

9. The system of any of claims 1-8, wherein the controller is adapted to periodically compare a net change in the first relative height to a first threshold and the net change in the second relative height to a second threshold and control a user interface to generate user feedback responsive to exceeding the threshold.

10. The system of any of claims 1-9, wherein the controller is adapted to compute a correction of the tissue pressure signal based on the function:

$$P_c\,(t) = P_0(t) + \Gamma(t)$$

where $\Gamma(t) = \alpha\Delta H1(t) + \beta\Delta H2(t)$
where P_c (t) is the corrected tissue pressure measurement, P_0 (t) is the original tissue pressure measurement, $\Gamma(t)$ is the correction parameter, $\alpha$ is a constant proportional to a density of the fluid contained in the pad, $\Delta H1(t)$ is the net change in the first relative height at time t, $\beta$ is a constant proportional to the density of blood, and $\Delta H2(t)$ is the net change in the second relative height at time *t.*

**11.** The system of claim 9, wherein $\alpha$ = 0.69 mmHg / cm height difference, and wherein $\beta$ = 0.78 mmHg / cm height difference.

**12.** The system of any of claims 1-11,

wherein the pressure transducer unit, cuff and/ or reference unit comprises an inertial measurement unit adapted to output an acceleration signal;
wherein the controller is further adapted to detect motion events of the pressure transducer unit, cuff and/or reference unit using the acceleration signal output from the inertial measurement unit;
wherein the controller is adapted to generate user feedback through control of a user interface responsive to the detection.

**13.** The system of claim 12,

wherein the controller is further adapted to determine a severity of each detected motion event based on a feature of the acceleration signal corresponding to the event;
wherein the controller is adapted to classify a measurement quality of a blood pressure measurement obtained over a time period which coincided with the motion event,
wherein the quality classification is determined based on a pre-determined mapping between motion event severity and measurement quality.

**14.** The system of any of claims 1-13,
wherein the height sensing subsystem includes a fluid line connected between the height reference unit and the pressure transducer unit and wherein the change in the relative height between the reference unit and the pressure transducer unit is computed based on a change in a pressure differential between the reference unit and the pressure transducer unit.

**15.** The system of any of claims 1-14,
wherein the system comprises a base unit, wherein the base unit houses the controller, wherein the base unit comprises a user interface including a display device, wherein the base unit includes a hemodynamic measurement module for computing one or more hemodynamic measurements based on the tissue pressure signal.

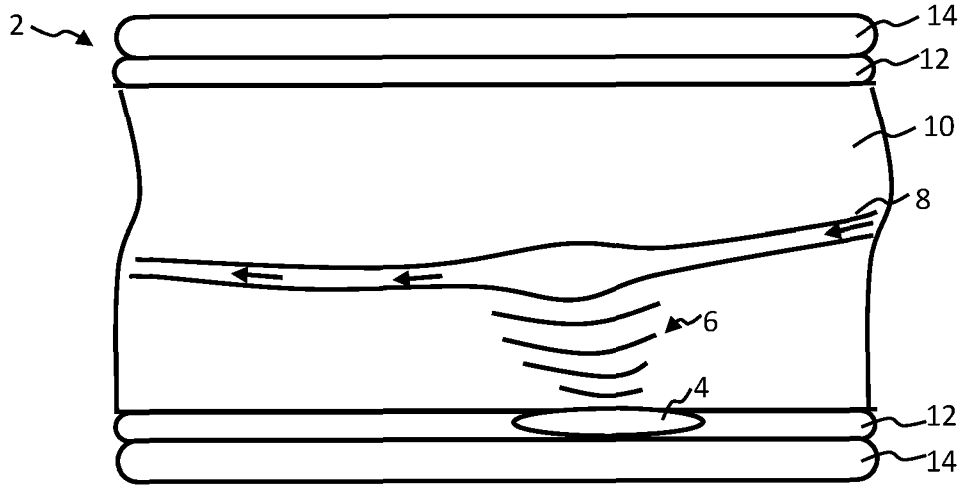


FIG. 1

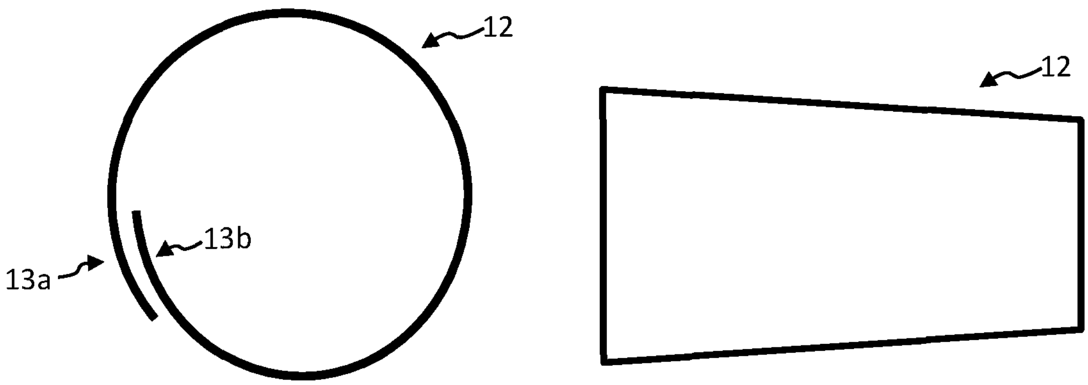


FIG. 2

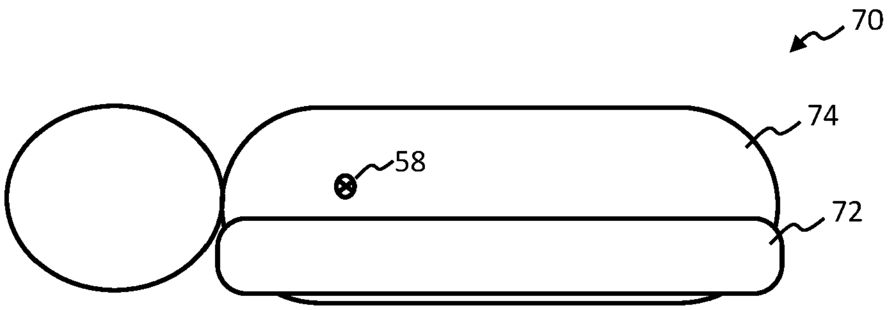


FIG. 3

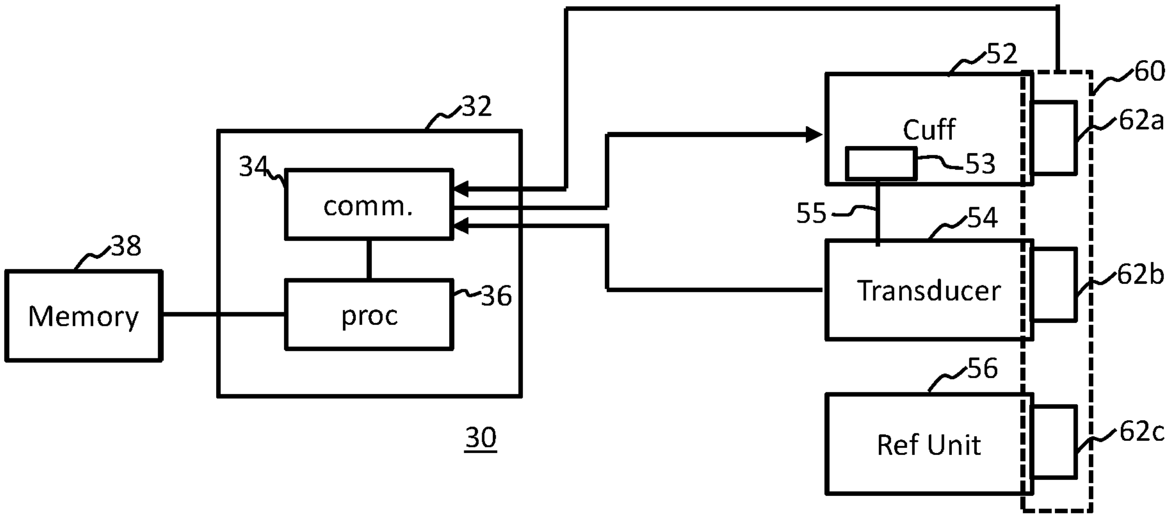


FIG. 4

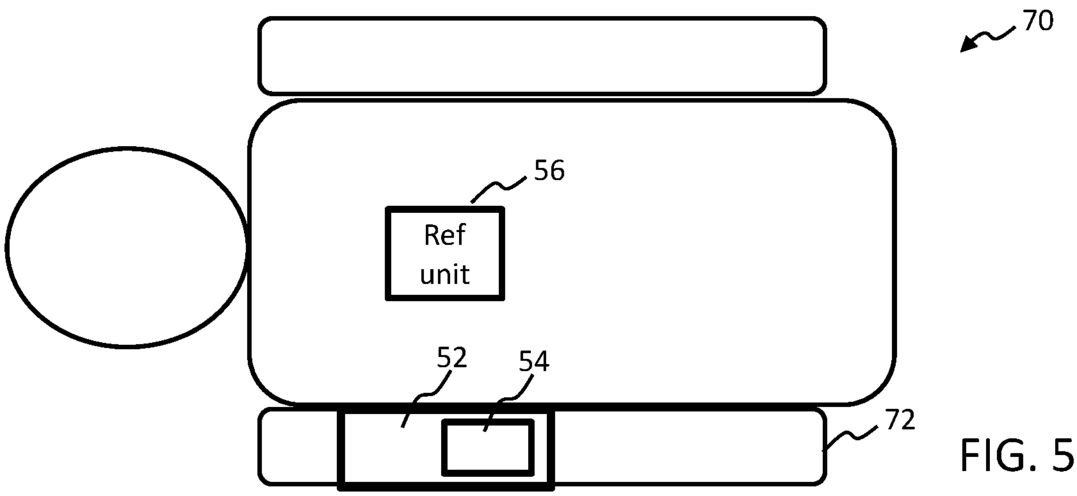


FIG. 5

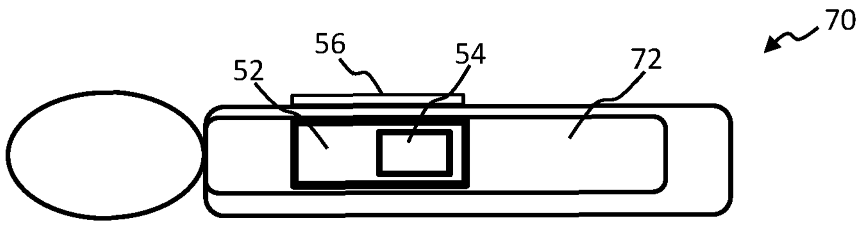


FIG.6

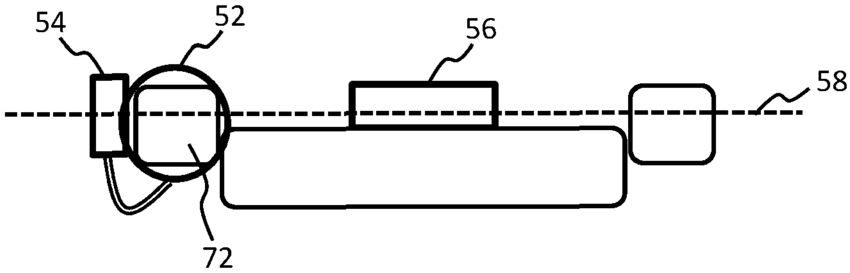


FIG. 7

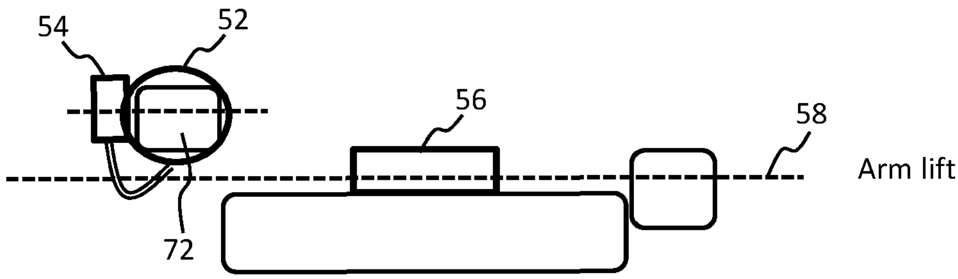


FIG. 8

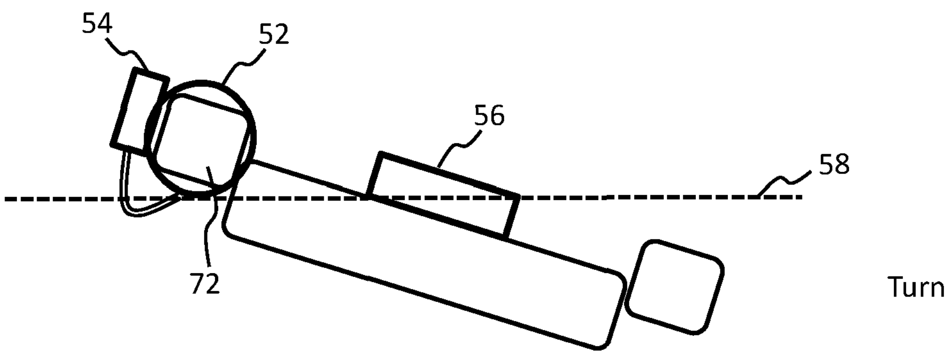


FIG. 9

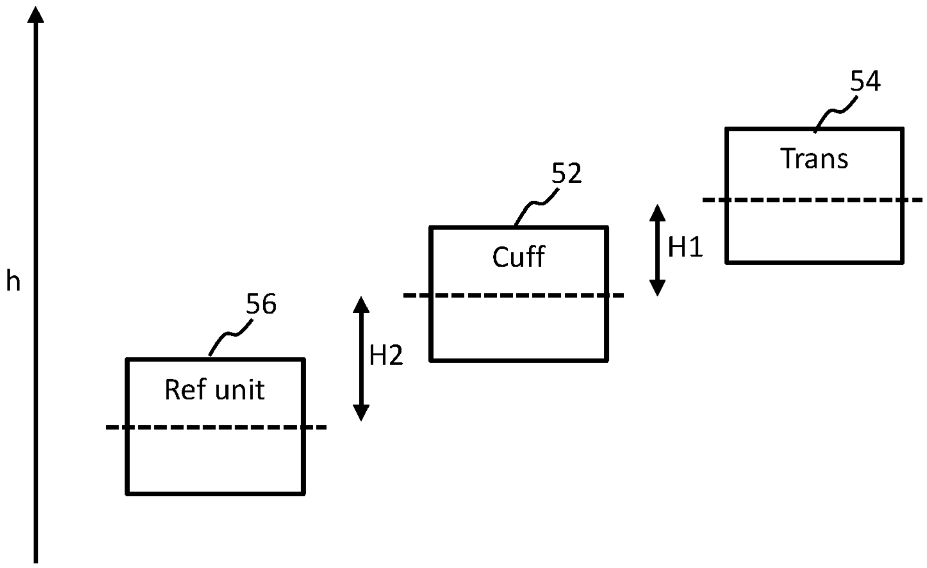


FIG. 10

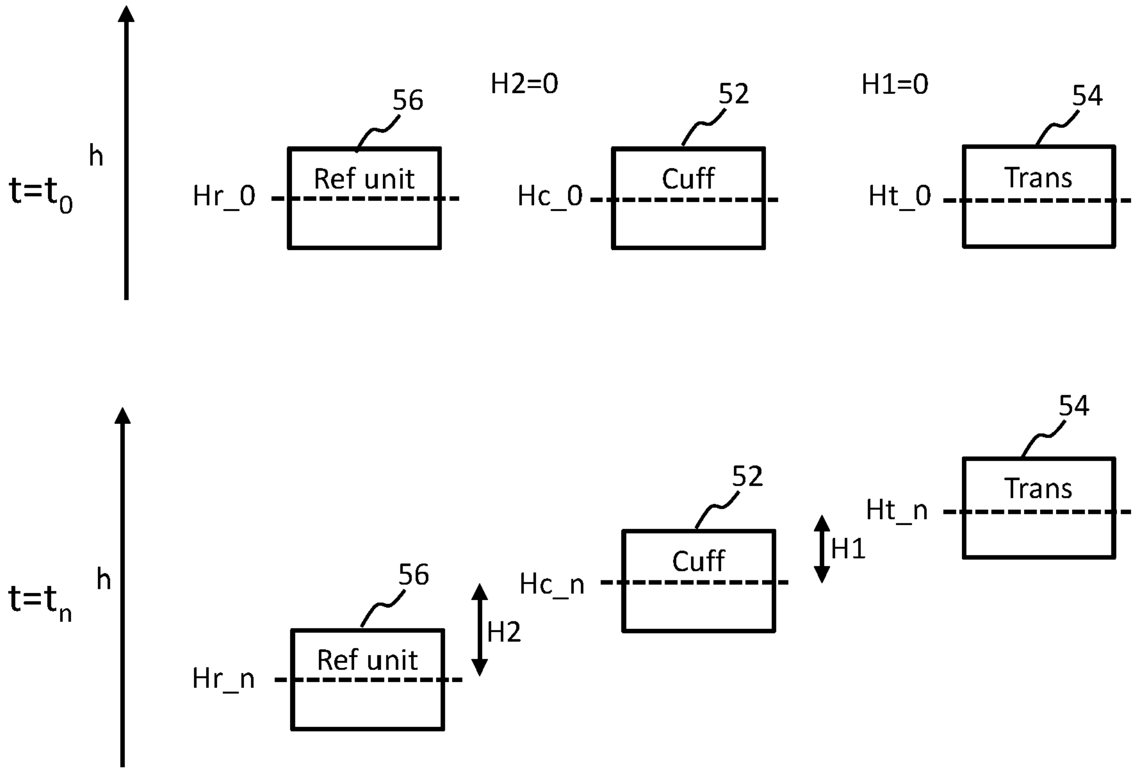


$\Delta H2 = \Delta Hc - \Delta Hr = [Hc_n - Hc_0] - [Hr_n - Hr_0]$

$\Delta H1 = \Delta Hc - \Delta Ht = [Hc_n - Hc_0] - [Ht_n - Ht_0]$

FIG. 11

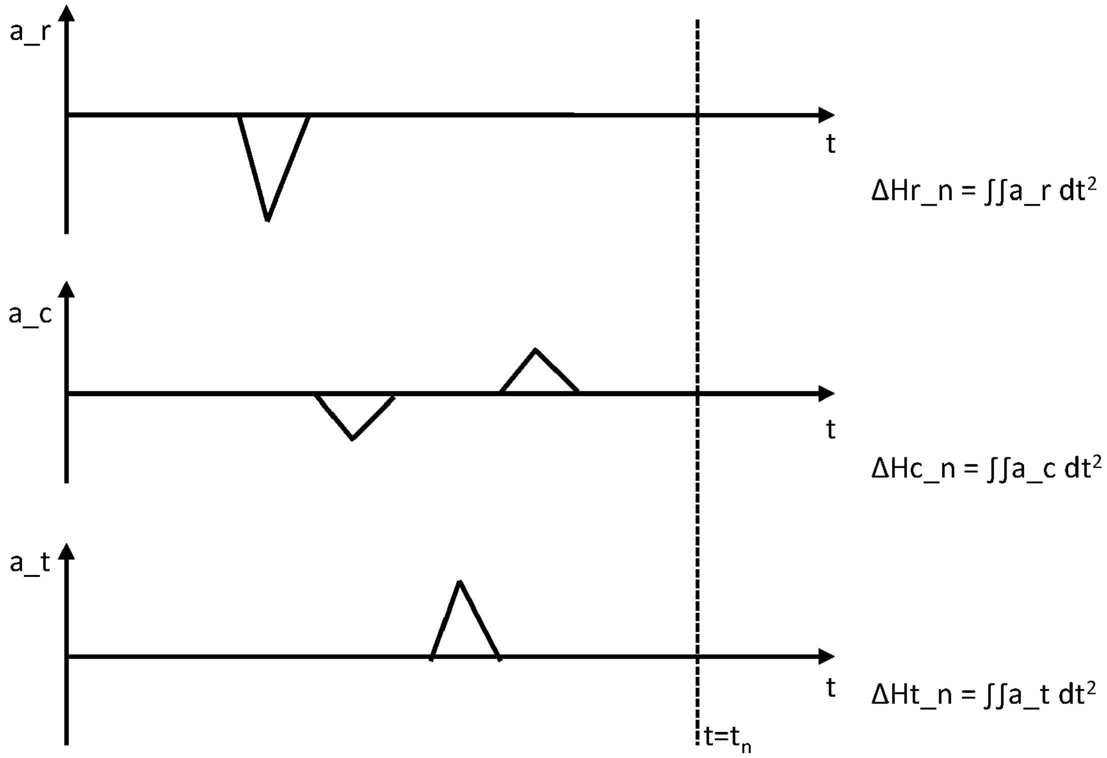


FIG. 12

Europäisches Patentamt
European Patent Office
Office européen des brevets

# EUROPEAN SEARCH REPORT

Application Number
EP 23 18 1942

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2006/036184 A1 (TENZER NATHAN J [US] ET AL) 16 February 2006 (2006-02-16)<br>* abstract; paragraph [0010] - paragraph [0020]; claims 1,10; figures 1-4 * | 1-6,8,9,12,14,15<br>7,8,10,13 | INV.<br>A61B5/021<br>A61B5/022<br>A61B5/00 |
| X<br>Y | US 2019/343407 A1 (HUIJBREGTS LAURENTIA JOHANNA [NL] ET AL) 14 November 2019 (2019-11-14)<br>* abstract; paragraph [0076] - paragraph [0157]; claim 6; figures 1-4 * | 1-6,9,12,14,15<br>7,10,11,13 | |
| Y<br>A | US 4 771 792 A (SEALE JOSEPH B [US]) 20 September 1988 (1988-09-20)<br>* abstract; column 2, line 38 - column 59, line 45; figures 1-15 * | 7,8,10,11,13<br>1-6,14,15 | |
| A | US 5 957 853 A (GIUFFRE KENNETH A [US]) 28 September 1999 (1999-09-28)<br>* abstract; claim 1; figures 1-2 * | 1-15 | TECHNICAL FIELDS SEARCHED (IPC)<br>A61B |
| A | WO 2005/032364 A1 (MONDO MEDICAL LTD [AU]; PARKIN JEOFF [AU]; BOYD CLIVE [AU]) 14 April 2005 (2005-04-14)<br>* abstract; figures 1-4 * | 1-15 | |

The present search report has been drawn up for all claims

1

| Place of search | Date of completion of the search | Examiner |
|---|---|---|
| The Hague | 31 July 2023 | Munteh, Louis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 18 1942

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2023

| Patent document cited in search report | Publication date | Patent family member(s) | Publication date |
|---|---|---|---|
| US 2006036184 A1 | 16-02-2006 | CA 2576235 A1 | 23-02-2006 |
| | | EP 1778079 A2 | 02-05-2007 |
| | | JP 2008509750 A | 03-04-2008 |
| | | US 2006036184 A1 | 16-02-2006 |
| | | WO 2006020917 A2 | 23-02-2006 |
| US 2019343407 A1 | 14-11-2019 | CN 110049718 A | 23-07-2019 |
| | | EP 3551059 A1 | 16-10-2019 |
| | | US 2019343407 A1 | 14-11-2019 |
| | | WO 2018104337 A1 | 14-06-2018 |
| US 4771792 A | 20-09-1988 | NONE | |
| US 5957853 A | 28-09-1999 | NONE | |
| WO 2005032364 A1 | 14-04-2005 | NONE | |

EPO FORM P0459

For more details about this annex : see Official Journal of the European Patent Office, No. 12/82